# EUROPEAN PATENT APPLICATION

(11) **EP 4 585 147 A1**
(43) Date of publication of application: **16.07.2025**
(21) Application number: 24156017.6
(22) Date of filing: 06.02.2024
(51) Int. Cl.: A61B 5/15, A61B 5/151

(54) **SINGLE-HANDED OPERABLE INFANT HEEL BLOOD SAMPLING DEVICE AND ITS SAFETY MECHANISM**

(30) Priority: 12.01.2024 CN 202410051464
(71) Applicant: Ningbo Medsun Medical Co., Ltd., Ningbo, Zhejiang 315034 (CN)
(72) Inventor: CHEN, Zhicheng, Ningbo, Zhejiang, 315034 (CN); CHEN, Kailiang, Ningbo, Zhejiang, 315034 (CN)
(74) Representative: Ipside

(57) **Abstract**

The invention discloses a single-handed operable infant heel blood sampling device and its safety mechanism, including a push-pull rod and an actuating rod partially extending outside the blood sampler housing; wherein the push-pull rod has a first hook portion and a buckle portion on its rod body; the actuating rod has a second hook portion that can engage with the first hook portion; to perform blood sampling, push down the push-pull rod to disengage the buckle portion from the housing, causing the first hook portion to engage with the second hook portion on the first end of the actuating rod; then pull up the push-pull rod to pull the actuating rod, and the actuating rod thus releases a pre-compressed spring set inside the blood sampler housing, allowing the blade originally positioned in a first position inside the housing to slide out from a cutting port and then be retracted into a second position inside the housing.

## Description

### Technical Field

This invention relates to a blood sampling device, particularly to a safety mechanism for an infant heel blood sampling device, and the infant heel blood sampling device itself.

### Background of the Invention

The usage method of a heel blood sampling device involves first releasing the safety mechanism and then grasping the infant's foot to collect blood. Existing heel blood sampling devices require a two-handed operation, where the safety mechanism needs to be pulled or twisted off before proceeding to grasp the infant's foot for blood sampling. The removal of the safety mechanism results in independent parts, generating unnecessary medical waste.

Therefore, professionals in this field are directed to developing an integrated infant heel blood sampling device that can be operated with a single hand.

### Summary of the Invention

To achieve the above objectives, the present invention first provides a single-handed releasable safety mechanism for an infant heel blood sampling device, comprising a push-pull rod and an actuating rod partially extending outside the blood sampler housing; wherein the push-pull rod has a first hook portion and a buckle portion on its rod body; the actuating rod has a second hook portion that can engage with the first hook portion; when the heel blood sampling device is in an unused state, the buckle portion of the push-pull rod engages with the blood sampler housing, and the first hook portion and a first end of the actuating rod are detached; wherein the above components and mechanisms are configured such that to perform blood sampling, pushing down the push-pull rod to disengage the buckle portion from the housing, causing the first hook portion to engage with the second hook portion on the first end of the actuating rod; then pulling up the push-pull rod to pull the actuating rod, and the actuating rod thus releases a pre-compressed spring set inside the blood sampler housing, allowing the blade originally positioned in a first position inside the housing to slide out from a cutting port and then be retracted into a second position inside the housing.

The present invention further provides a single-handed operable infant heel blood sampling device, comprising a housing, and a push-pull rod and an actuating rod partially extending outside the housing, with a cutting port on the housing; wherein inside the housing, there are a first slot and a second slot, as well as a blade slider and a blade handle; the blade slider is slidable in the first slot; a first end of the blade handle is rotatably mounted on the blade slider, and a second end of the blade handle is slidable in the second slot; a blade is fixedly mounted on the blade handle; inside the housing, there is also a first pivot; a first end of the actuating rod extends outside the housing; a second end of the actuating rod is rotatably mounted on the first pivot; there is a protrusion on the second end of the actuating rod; a limiting portion is disposed inside the housing; wherein when the heel blood sampling device is in an unused state, a spring cooperating with the blade slider is held in a pre-compressed state through the cooperation of the protrusion and the limiting portion, at which point the blade is in a first position retracted inside the housing; in the middle of the actuating rod, there is a second pivot; a rotating end of the push-pull rod is rotatably mounted on the second pivot; the push-pull rod has a first hook portion and a buckle portion on its rod body; there is a second hook portion on the first end of the actuating rod that can cooperate with the first hook portion; wherein when the heel blood sampling device is in an unused state, the buckle portion of the push-pull rod engages with the housing, and at this time, the first hook portion of the push-pull rod and the first end of the actuating rod are detached; wherein the components and mechanisms above are configured as such that to perform blood sampling, first push down the push-pull rod, causing the buckle portion to disengage from the housing, and the first hook portion to engage with the second hook portion on the first end of the actuating rod; then pull up the push-pull rod to further pull the actuating rod, and thus the lever force of the actuating rod overcomes the blocking of the limiting portion, causing the actuating rod to rotate, and the pre-compressed spring is released; then the blade slider, driven by the spring, slides to the other side in the first slot; thus the first end of the blade handle rotates while sliding with the blade slider, and the second end of the blade handle slides in the second slot; these movements cause the blade to slide out from the cutting port and then be retracted into the second position inside the housing.

Furthermore, a damping portion is further disposed in the housing, and a damping mating portion on the actuating rod is disposed to cooperate with the damping portion.

Furthermore, the protrusion is set to be destroyed when overcoming the obstruction of the limiting portion.

Furthermore, the blade slider is slidable in the first slot through the cooperation of a column thereon and the first slot.

Furthermore, a pin is disposed on the blade slider, and the first end of the blade handle is rotatably mounted on the pin of the blade slider.

Furthermore, the second end of the blade handle has a sliding portion; the sliding portion is set in the second slot inside the housing.

The present invention further provides a method for releasing the single-handed releasable safety mechanism for an infant heel blood sampling device as described above, using one hand to first push down the push-pull rod, causing the buckle portion to disengage from the housing and further causing the first hook portion thereon to engage with the second hook portion on the first end of the actuating rod, and then pulling up the push-pull rod.

The present invention further provides a method for using the single-handed operable infant heel blood sampling device as described above, using one hand to grasp the infant's heel and the other hand to first push down the push-pull rod, causing the buckle portion to disengage from the housing and further causing the first hook portion thereon to engage with the second hook portion on the first end of the actuating rod, and then pulling up the push-pull rod to release the safety mechanism of the infant heel blood sampling device and then proceed with blood sampling.

The present invention provides a single-handed operable infant heel blood sampling device and its safety mechanism. In addition to being operable with one hand, the entire heel blood sampling device remains integrated after blood collection, so as to avoid unnecessary medical waste.

### Brief description of the Drawings

FIG. 1 provides an overall appearance diagram of an infant heel blood sampling device in a preferred embodiment of the present invention.
FIG. 2 is an internal structural diagram of the infant heel blood sampling device in Figure 1.
FIG. 3 is a partial exploded view of components in Figure 2.
FIG. 4 is an internal structural front view of the infant heel blood sampling device in Figure 1 when not in use;
FIG. 5 is the internal structural front view of the infant heel blood sampling device in Figure 1 during the first step of releasing the safety mechanism;
FIG. 6 is the internal structural front view of the infant heel blood sampling device in Figure 1 during the second step of releasing the safety mechanism;
FIG. 7 is the internal structural front view of the infant heel blood sampling device in Figure 1 after releasing the safety and completing the blood collection.

### Detailed Description of Embodiments

A plurality of preferred embodiments of the present invention are described below with reference to the drawings, which makes its technical content more clear and convenient to understand. The present invention may be embodied in many different forms of embodiments, and the scope of protection of the present invention is not limited to the embodiments set forth herein.

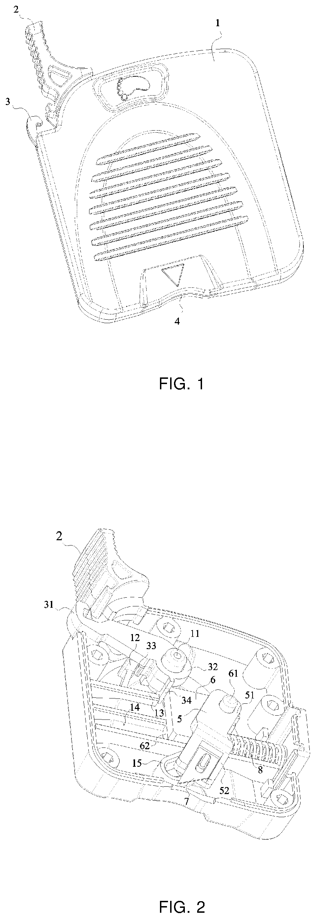

According to a preferred embodiment of the infant heel blood sampling device of the present invention, the overall appearance is shown in Figure 1. It shows that the heel blood sampling device includes a housing 1 and push-pull rod 2 and actuating rod 3 partially extending outside the housing 1. The housing 1 also has a cutting port 4.

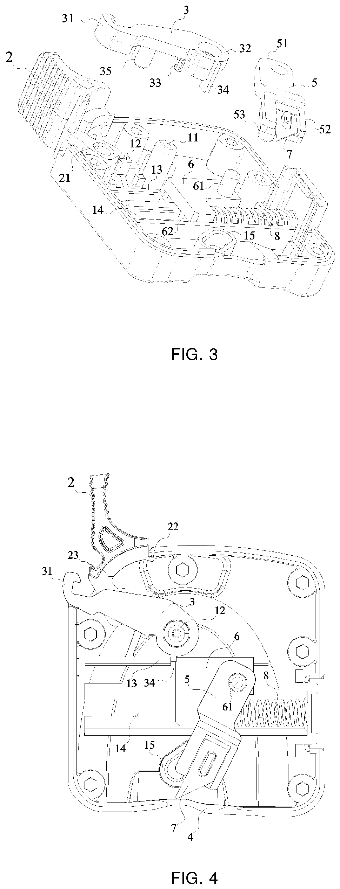

The internal structure of the heel blood sampling device is shown in Figures 2 and 3, where the device is in an unused state. Inside the housing 1, there is a first slot 14. The blade slider 6, through the cooperation of its column 62 and the first slot 14, can slide in the first slot 14. The blade slider 6 also has a pin 61. The first end 51 of the blade handle 5 is rotatably mounted on the pin 61 of the blade slider 6, and the second end 52 of the blade handle 5 is fixedly equipped with a blade 7. The second end 52 of the blade handle 5 also has a sliding portion 53. The sliding portion 53 is set in the second slot 15 inside the housing 1.

Inside the housing 1, there is also a first pivot 11. The first end 31 of the actuating rod 3 extends outside the housing 1, and the second end 32 is rotatably mounted on the first pivot 12. The second end 32 of the actuating rod 3 has a protrusion 34. A limiting part 13 is set inside the housing 1. Also referring to Figures 2-4, when the heel blood sampling device is in the unused state, the spring 8 cooperating with the blade slider 6 is held in a pre-compressed state through the cooperation of the protrusion 34 and the limiting part 13. At this time, the blade 7 is in the first position retracted inside the housing 1.

Referring also to Figures 2-4, a second pivot 35 is similarly set in the middle of the actuating rod 3. The rotating end 21 of the push-pull rod 2 is rotatably mounted on the second pivot 35. The push-pull rod 2 has a first hook portion 23 and a buckle portion 22 on it. A second hook portion, compatible with the first hook portion 23, is set on the first end 31 of the actuating rod 3. In the unused state, the buckle portion 22 of the push-pull rod engages on the housing 1, and at this time, the first hook portion 23 and the first end 31 of the actuating rod 3 are detached.

When the heel blood sampling device is in the unused state as shown in Figures 2-4, even if the actuating rod 3 is moved, the spring 8 will not be released due to the engagement of the protrusion 34 and the limiting part 13. As a result, the blade 7 remains retracted inside the housing 1.

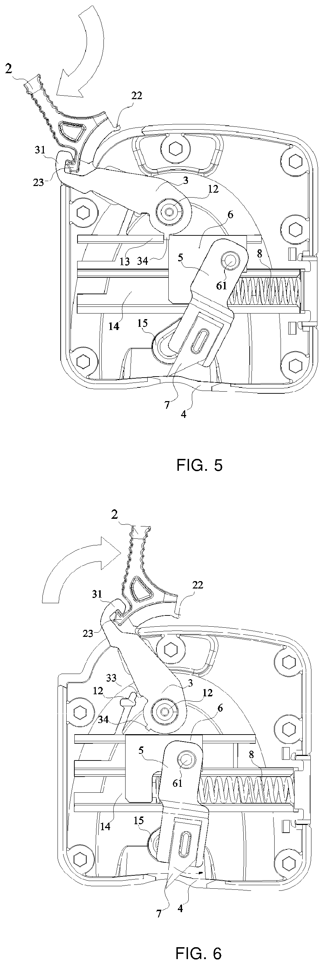

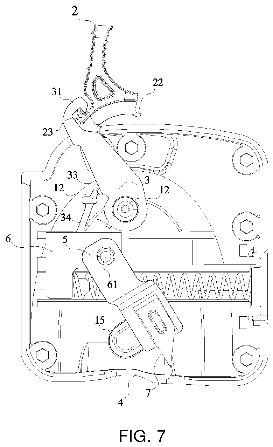

Next, referring to Figures 5-7, when using the heel blood sampling device for blood collection, it is necessary to first, as indicated by the arrow in Figure 5, push down the push-pull rod 2, causing the buckle portion 22 to disengage from the housing 1, while engaging the second hook portion on the first end 31 of the actuating rod 3 with the first hook portion 23. Then, as indicated by the arrow in Figure 6, pull up the push-pull rod 2. The push-pull rod 2 pulls the actuating rod 3, and its leverage force allows the protrusion 34 to overcome the resistance of the limiting portion 13, causing the actuating rod to rotate, and releasing the pre-compressed spring 8. The blade slider 6, driven by the spring 8, slides to the other side in the first slot 14. The first end 51 of the balde handle 5, while sliding with the blade slider 6, rotates around the pin 61, and the second end 53 slides in the second slot 15 through the sliding part 53, resulting in the final trajectory that allows the blade 7 on it to slide out of the cutting port 4 and be retracted into the second position inside the housing 1. In some embodiments, the overcoming of the resistance of the limiting part 13 may destroy the protrusion 34, thereby avoiding the reuse of the blood collector.

In this embodiment, there is also a damping portion 12 inside the housing 1. On the actuating rod 3, there is a damping mating portion 33 that can engage the damping part 12. The damping portion 12 and the damping mating portion 33 can provide better damping feedback to the hand when pulling the push-pull rod 2.

The foregoing detailed description describes preferred embodiments of the invention. It should be understood that many modifications and variations can be made in accordance with the concepts of the present invention without creative efforts by those of ordinary skill in the art. Accordingly, all the modifications and alterations of the device and method made by those skilled in the art without departing from the spirit shall be deemed as still within the scope of the invention as defined by the appended claims.

## Claims

1. A single-handed releasable safety mechanism for an infant heel blood sampling device, **characterized by** comprising a push-pull rod (2) and an actuating rod (3) partially extending outside the blood sampler housing (1);
wherein the push-pull rod (2) has a first hook portion (23) and a buckle portion (22) on its rod body; the actuating rod (3) has a second hook portion that can engage with the first hook portion (23); when the heel blood sampling device is in an unused state, the buckle portion (22) of the push-pull rod (2) engages with the blood sampler housing (1), and the first hook portion (23) and a first end (31) of the actuating rod (3) are detached;
wherein the above components and mechanisms are configured such that to perform blood sampling, pushing down the push-pull rod (2) to disengage the buckle portion (22) from the housing (1), causing the first hook portion (23) to engage with the second hook portion on the first end (31) of the actuating rod (3); then pulling up the push-pull rod (2) to pull the actuating rod (3), and the actuating rod (3) thus releases a pre-compressed spring set inside the blood sampler housing (1), allowing the blade (7) originally positioned in a first position inside the housing (1) to slide out from a cutting port (4) and then be retracted into a second position inside the housing (1).

2. A single-handed operable infant heel blood sampling device, **characterized by** comprising a housing (1), and a push-pull rod (2) and an actuating rod (3) partially extending outside the housing (1), with a cutting port (4) on the housing (1);
wherein inside the housing (1), there are a first slot (14) and a second slot (15), as well as a blade slider (6) and a blade handle (5); the blade slider (6) is slidable in the first slot (14); a first end (51) of the blade handle (5) is rotatably mounted on the blade slider (6), and a second end (52) of the blade handle (5) is slidable in the second slot (15); a blade is fixedly mounted on the blade handle (5); inside the housing (1), there is also a first pivot (11); a first end (31) of the actuating rod (3) extends outside the housing (1); a second end (32) of the actuating rod (3) is rotatably mounted on the first pivot (11); there is a protrusion (34) on the second end (32) of the actuating rod (3); a limiting portion (13) is disposed inside the housing (1);
wherein when the heel blood sampling device is in an unused state, a spring cooperating with the blade slider (6) is held in a pre-compressed state through the cooperation of the protrusion (34) and the limiting portion (13), at which point the blade (7) is in a first position retracted inside the housing (1); in the middle of the actuating rod (3), there is a second pivot (35); a rotating end (21) of the push-pull rod (2) is rotatably mounted on the second pivot (35); the push-pull rod (2) has a first hook portion (23) and a buckle portion (22) on its rod body; there is a second hook portion on the first end (31) of the actuating rod (3) that can cooperate with the first hook portion (23);
wherein when the heel blood sampling device is in an unused state, the buckle portion (22) of the push-pull rod (2) engages with the housing (1), and at this time, the first hook portion (23) of the push-pull rod (2) and the first end (31) of the actuating rod (3) are detached;
wherein the components and mechanisms above are configured as such that to perform blood sampling, first push down the push-pull rod (2), causing the buckle portion (22) to disengage from the housing (1), and the first hook portion (23) to engage with the second hook portion on the first end (31) of the actuating rod (3); then pull up the push-pull rod (2) to further pull the actuating rod (3), and thus the lever force of the actuating rod (3) overcomes the blocking of the limiting portion (13), causing the actuating rod (3) to rotate, and the pre-compressed spring is released; then the blade slider (6), driven by the spring, slides to the other side in the first slot (14); thus the first end (51) of the blade handle (5) rotates while sliding with the blade slider (6), and the second end (52) of the blade handle (5) slides in the second slot (15); these movements cause the blade (7) to slide out from the cutting port (4) and then be retracted into the second position inside the housing (1).

3. The single-handed operable infant heel blood sampling device as claimed in claim 1, wherein a damping portion (12) is further disposed in the housing (1), and a damping mating portion (33) on the actuating rod (3) is disposed to cooperate with the damping portion (12).

4. The single-handed operable infant heel blood sampling device as claimed in claim 1, wherein the protrusion (34) is set to be destroyed when overcoming the obstruction of the limiting portion (13).

5. The single-handed operable infant heel blood sampling device as claimed in claim 1, wherein the blade slider (6) is slidable in the first slot (14) through the cooperation of a column (62) thereon and the first slot (14).

6. The single-handed operable infant heel blood sampling device as claimed in claim 1, wherein a pin (61) is disposed on the blade slider (6), and the first end (51) of the blade handle (5) is rotatably mounted on the pin (61) of the blade slider (6).

7. The single-handed operable infant heel blood sampling device as claimed in claim 6, wherein the second end (52) of the blade handle (5) has a sliding portion (53); the sliding portion (53) is set in the second slot (15) inside the housing (1).

8. A method for releasing the single-handed releasable safety mechanism for an infant heel blood sampling device as claimed in claim 1, **characterized by** using one hand to first push down the push-pull rod (2), causing the buckle portion (22) to disengage from the housing (1) and further causing the first hook portion (23) thereon to engage with the second hook portion on the first end (31) of the actuating rod (3), and then pulling up the push-pull rod (2).

9. A method for using the single-handed operable infant heel blood sampling device as claimed in claim 2, **characterized by** using one hand to grasp the infant's heel and the other hand to first push down the push-pull rod (2), causing the buckle portion (22) to disengage from the housing (1) and further causing the first hook portion (23) thereon to engage with the second hook portion on the first end (31) of the actuating rod (3), and then pulling up the push-pull rod (2) to release the safety mechanism of the infant heel blood sampling device and then proceed with blood sampling.
